# EUROPEAN PATENT APPLICATION

(11) **EP 3 835 403 A1**
(43) Date of publication of application: **16.06.2021**
(21) Application number: 19215115.7
(22) Date of filing: 11.12.2019
(51) Int. Cl.: C12M 1/00, C12M 1/26

(54) **AUTOMATED MEDIUM EXCHANGE STRATEGY FOR SUSPENSION CELLS**

(71) Applicant: Sartorius Stedim Biotech GmbH, 37079 Göttingen (DE); Repairon GmbH, 37079 Göttingen (DE)
(72) Inventor: Haupt, Luis, 37083 Göttingen (DE); Hupfeld, Julia, 37139 Adelebsen (DE)
(74) Representative: Schiweck Weinzierl Koch Patentanwälte Partnerschaft mbB

(57) **Abstract**

The present invention relates to a method of changing culture medium of a suspension culture, the suspension culture comprising cells suspended in the culture medium, the method comprising: (i) transferring a fraction of the suspension culture into a container, wherein the container comprises at least one opening at the bottom; (ii) allowing the cells comprised in the fraction of the suspension to settle at the at least one opening at the bottom of the container by gravitation, thereby forming a supernatant; (iii) dispensing the cells settled at the bottom of the container (back) into the suspension culture; (iv) discarding the supernatant.

## Description

### TECHNICAL FIELD OF THE INVENTION

The present invention relates to a method of changing culture medium of a suspension culture, the suspension culture comprising cells suspended in the culture medium.

### BACKGROUND

Induced pluripotent stem cells (iPSC) and other adherent cells are traditionally cultivated and expanded in static cell culture vessels ("2D culture"). Due to their adherent properties, this form of cultivation allows an easy medium change: used medium is removed, while the adherent cells remain on the surface of the cell culture vessel. However, 2D culture is only scalable and automated to a limited extent, which results in a high manual and cost-intensive workload, for example to produce the large number of cells for clinical therapies and commercial therapeutic cell products under GMP conditions.

With regard to commercial bioprocess development, the cultivation in suspension culture in stirred bioreactors is an attractive alternative to traditional 2D culture. E.g., the cultivation of iPSC in suspension in the form of iPSC aggregates ("3D culture") has been described several times in the literature (Olmer et al. 2012; Kwok et al. 2018; Amit et al. 2010). The 3D suspension culture offers the possibility to develop an efficient, reproducible and scalable bioprocess in which culture parameters such as pH, dissolved oxygen (DO) and temperature can be controlled.

In contrast to traditional 2D cultivation, changing media in suspension cultures remains a challenge because the cells/cell aggregates have to be separated from the medium and retained while the used medium is removed. In bioprocessing this can be solved in the classical way by perfusion. Another possibility already described is to interrupt the stirring and sedimentation of the cells/cell aggregates, followed by the removal of the medium and the addition of fresh medium (see e.g. Kwok et al. 2018). However, prolonged interruption of stirring may lead to the formation of larger aggregates (aggregate fusion). This leads to the formation of larger, inhomogeneous aggregates, which in turn leads to differences in mass transfer and signal gradients as well as to spontaneous differentiation and decrease of pluripotency in the cell aggregates (Lipsitz et al. 2018, see also Example 1).

Accordingly, there is still a need for methods of changing culture medium of a suspension culture, in particular in which the entire process can be performed without removing the cells or aggregates from the system and without exposing the cells to the stress that is associated with sedimentation and/or centrifugation for extended periods of time. The present invention aims to address this need.

### SUMMARY OF THE INVENTION

This need is solved by the subject-matter as defined in the claims. It is presented herein a method of changing culture medium of a suspension culture, the suspension culture comprising cells suspended in the culture medium.

Accordingly, the present invention relates to a method of changing culture medium of a suspension culture, the suspension culture comprising cells suspended in the culture medium, the method comprising:
(i) transferring a fraction of the suspension culture into a container, wherein the container comprises at least one opening at the bottom;
(ii) allowing the cells comprised in the fraction of the suspension to settle at the at least one opening at the bottom of the container by gravitation, thereby forming a supernatant;
(iii) dispensing the cells settled at the bottom of the container (back) into the suspension culture;
(iv) discarding the supernatant.

The method may be carried out in a bioreactor. The bioreactor may be a stirred bioreactor (STR), a rocking motion bioreactor (RM) and/or a multiparallel bioreactor.

The suspension culture may be continuously stirred.

The cells may be essentially homogenously distributed in the culture medium.

The container may be tubular. The container may have a conical bottom. The container may be a pipette tip, a (single-use) bag or the conical/conically-shaped part of a (single-use) bag.

The fraction of the suspension culture may be aspirated into the container in step (i).

The cells may be eukaryotic cells, fungal cells such as yeast cells such as *P. pastoris,* insect cells such as *Drosophila melanogaster* S2 and *Spodoptera frugiperda* Sf9 cells, bacterial cells such as *E. coli, Streptomyces* and *Salmonella typhimurium* cells, or plant cells. Preferably, the cells are eukaryotic cells.

The cells may be adherent cells that are cultured in suspension.

The cells may be selected from the group consisting of, primary cells, cells obtained from a tissue or an organ, immortalized cells, pluripotent stem cells, preferably the cells are pluripotent stem cells, preferably induced pluripotent stem cells (iPSC), or iPSC-derived cells. The cells may be selected from the list consisting of TC-1133, the Human Episomal iPSC Line of Gibco ATCC ACS-1004, ATCC ACS-1021, ATCC ACS-1025, ATCC ACS-1027, ATCC ACS-1030, HEK293, HEK293T, BHK 21, CHO, NS0, and Sp2/0-Ag14. The cells may be human or non-human.

The cells may be cell aggregates. The cell aggregates may have an average diameter between about 50 and 800 µm, between about 150 and 800 µm, of at least about 800 µm, of at least about 600 µm, of at least about 500 µm, of at least about 400 µm, of at least about 300 µm, of at least about 200 µm, of at least about 150 µm, between about 300 and 500 µm, between about 150 and 300 µm, between about 50 and 150 µm, between about 80 to 100 µm, between about 180 to 250 µm or between about 200 to 250 µm.

The method may further comprise: (v) adding a volume equivalent to the supernatant to the suspension culture.

In step (ii) of the method of the invention, the period, in which the cells are allowed to settle, may be at least 1 min, at least 2 min, at least 3 min, at least 4 min, at least 5 min, at least 6 min, at least 7 min, at least 8 min, at least 9 min, at least 10 min, at least 11 min, at least 12 min, at least 13 min, at least 14 min, at least 15 min, at least 16 min, at least 17 min, at least 18 min, at least 19 min or at least 20 min.

At least 70%, at least 80%, at least 90%, at least 95%, at least 97.5%, at least 99% or essentially all cells of the cells transferred in step (i) may be dispensed (back) into the suspension culture in step (iii).

The cells may be grown on microcarrier particles. The suspension culture may be a microcarrier culture.

### BRIEF DESCRIPTION OF THE DRAWINGS

The invention will be better understood with reference to the detailed description when considered in conjunction with the non-limiting examples and the accompanying drawings, in which:

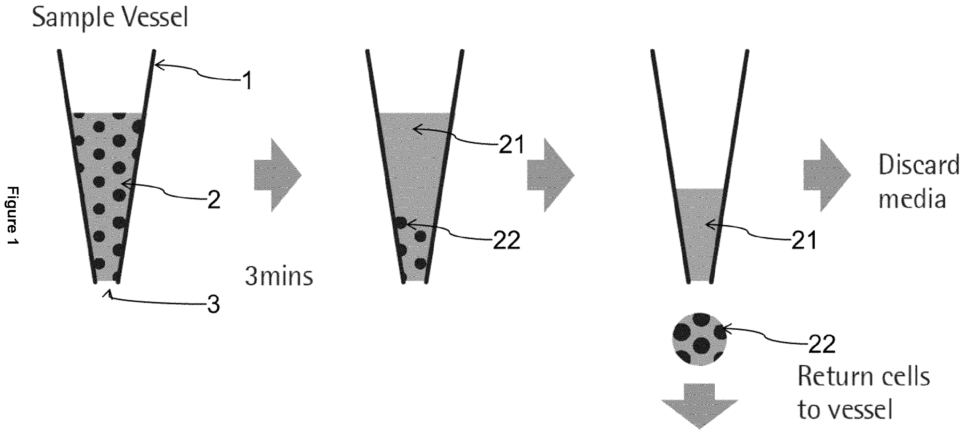

**Fig. 1** shows an exemplary embodiment of the method of the invention that can also be called "tip settling" or "pipette tip settling". A fraction of the suspension culture (2) has been transferred into a container (1) from the suspension culture. This may be an automated process, in which the tip of the container (1) comprising the at least one opening (3) is immersed into the suspension culture and a fraction of the suspension culture (2) is aspirated. The exemplary container (1), here a conical pipette tip shown in cross section, has one opening at the bottom (3).
   The cells (22) are than allowed to settle at the at least one opening at the bottom of the container by gravitation by holding the container, here the pipette tip, static for a defined period of time such as about 3 minutes or about 5 minutes. The sedimentation may take place while the container is still immersed or the container may be removed from the suspension culture again. Thereby, a supernatant of culture medium essentially free of cells is generated (21).
   After a sufficient number of cells or cell aggregates has sedimented, the cells (22) are dispensed back into the suspension culture while the supernatant of the culture medium (21) is retained. The remaining supernatant (21) may be discarded, e.g. by dispensing it into a waste container.
   The process shown in Fig. 1 may be continuously repeated. This allows a continuous replacement of the culture medium, which may be seen as mimicking a perfusion medium exchange process.
**Fig. 2** shows the morphology of iPSC aggregates. Depicted are images of wells of a 24-well plate containing aggregates that were sampled from ambr15 suspension cultures. The images were acquired using a Cellavista Cell Imager. Scale bars: 3 mm.
**Fig. 3** shows the iPSC expansion rate after 4 days.
**Fig. 4** shows the expression of pluripotency-related genes. Expression of pluripotency-related genes was analyzed with flow cytometry at day 4 of iPSC suspension culture.

### DETAILED DESCRIPTION OF THE INVENTION

The present invention is described in detail in the following and will also be further illustrated by the appended examples and figures.

Automated medium exchange of suspension cultures in a bioreactor remains a challenge. Manual medium exchange usually involves the transfer of at least a portion of the suspension culture out of the bioreactor and include, e.g., centrifugation of the cells. This mechanical stimulation can have negative effects on cell viability or functions such as unwanted differentiation of stem cells (Lipsitz et al. 2018, see also Example 1). The method of medium exchange of the invention avoids the transfer of cells out of the bioreactor as well as stressful mechanical stimulations. Thereby, the number of manual interactions is limited and the risk of contamination is limited.

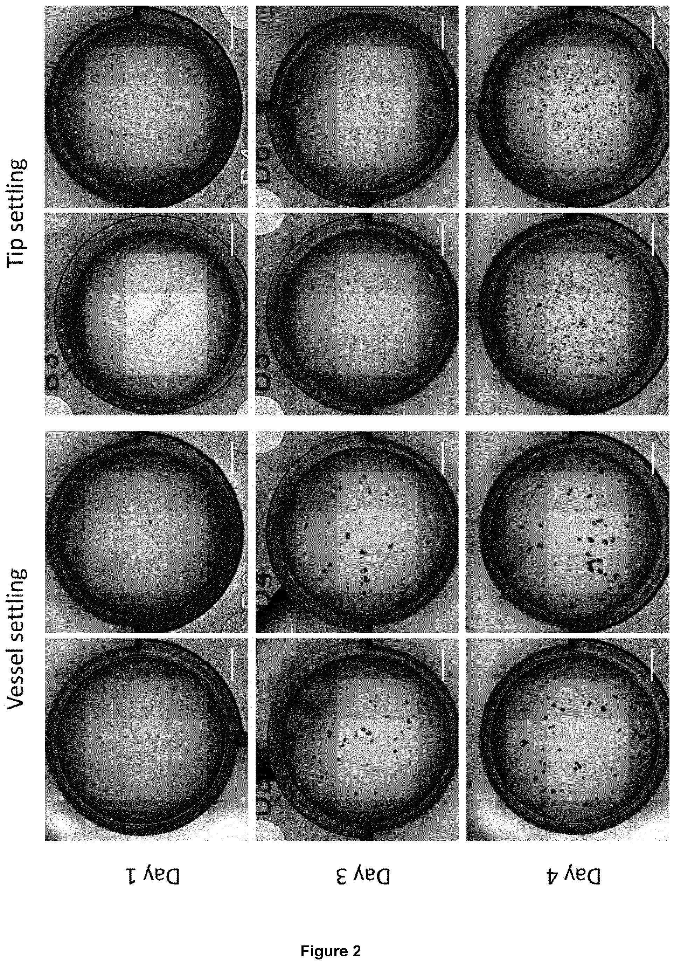

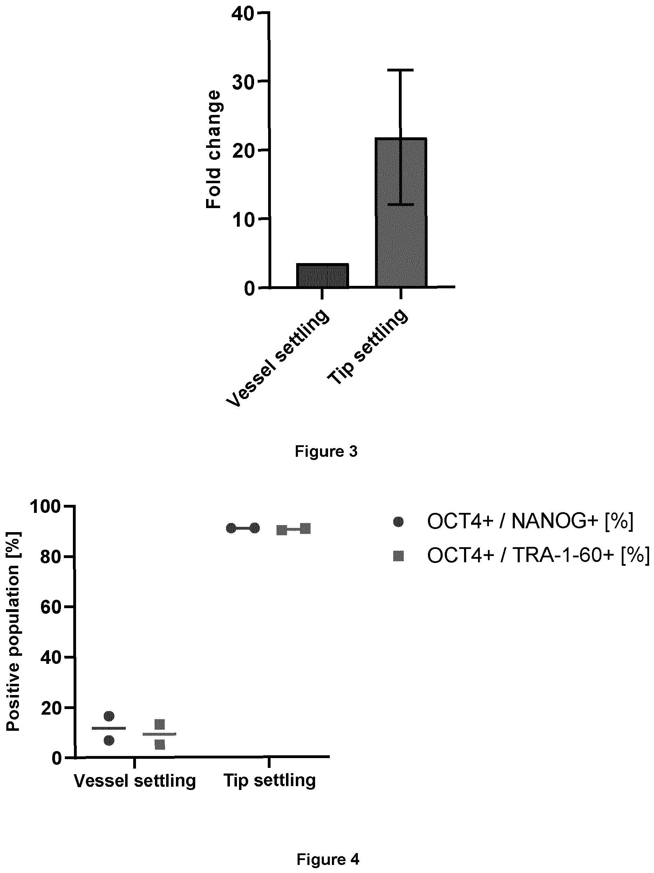

One possibility of automated medium exchange of a suspension culture in a bioreactor ("vessel settling") is stopping of the stirring and allowing the cells to settle at the bottom of the bioreactor. The supernatant can then be aspirated and be replaced with fresh medium. This however also leads to mechanical stimulation of the cells, which can lead to irregular growth and loss of pluripotency (see e.g. Example 1, Figures 2 and 4). This problem is overcome by the method of the present invention.

The problems outlined above are solved by the method of the present invention ("tip settling"). In a bioreactor, cells in suspension culture are preferably stirred. This is especially important for normally adherent cells that may form cell aggregates in suspension culture. In the state of the art, the stirring is stopped and the cells or cell aggregates are allowed to sediment. After sedimentation, the supernatant is exchanged and the stirring is started again (see e.g. Kwok et al. 2018). The sedimentation however has a negative impact on the cells (Lipsitz et al. 2018, Example 1, Figures 2 and 4). In the method of the present invention, the stirring of the bioreactor is thus preferably not stopped but runs continuously and the stopping of stirring preferably is avoided. However, the method of the invention can also be carried out in a bioreactor, in which the culture medium is not stirred.

Despite surprisingly yielding regularly formed/shaped cell aggregates (Figure 2) and a dramatically higher rate of iPSCs positive for pluripotency markers (Figure 4) in comparison to the vessel settling method, the method of the present invention ("tip settling") also leads to an increased growth rate of iPSCs as shown in Example 1 and Figure 4. In sum, the method of the present invention is an improved method of changing culture medium of a suspension culture.

In an exemplary embodiment of method of the invention (see also Fig. 1 or Example 1), a fraction of the cell culture medium is transferred in a container, e.g. a pipette tip. After transfer, the cells are allowed to settle at the bottom of the container by gravitation at the at least one opening in the bottom of the container. Thereby, the cells concentrate at the bottom of the fraction of the culture medium, i.e. a supernatant that preferably is essentially free of cells is formed. After sedimentation, the cells are dispensed back into the culture medium of the bioreactor, e.g. by ejecting the cells while the supernatant remains in the container. The supernatant can be discarded. To maintain a constant volume of culture medium in the bioreactor, the discarded supernatant may be replaced by an equal amount of fresh culture medium. Importantly, only a (relatively minor) part of the cells is taken from the suspension culture while the remaining cells are continuously stirred. Thus, only a small number of cells are in the container, which can reduce the time needed for medium exchange and therefore also reduce the time a cell is sedimented during medium exchange. Most importantly, it is not necessary to remove the cells from the bioreactor.

Accordingly, the present invention relates to a method of changing culture medium of a suspension culture, the suspension culture comprising cells suspended in the culture medium, the method comprising:
(i) transferring a fraction of the suspension culture into a container, wherein the container comprises at least one opening at the bottom;
(ii) allowing the cells comprised in the fraction of the suspension to settle at the at least one opening at the bottom of the container by gravitation, thereby forming a supernatant;
(iii) dispensing the cells settled at the bottom of the container (back) into the suspension culture;
(iv) discarding the supernatant.

"Dispensing" as used herein is not limited to applying a force from the inside of the container. Alternatively, also removing the cells from the outside of the container, e.g. by sucking out of the cells from the container, is envisioned by the present invention. While doing the, the supernatant may remain in the container and can be discarded afterwards.

The amount or volume of culture medium that is discarded may be replaced by adding a volume equivalent to the supernatant to the suspension culture. Thereby, the volume of the suspension culture can be kept constant. Accordingly, the method of the present invention may further comprise step (v): adding a volume equivalent to the supernatant to the suspension culture. The method of the present invention may also be used to increase or decrease the volume of the suspension culture. E.g., by adding a volume greater than the volume of the supernatant to the suspension culture, the (total) volume of the suspension culture is increased. Accordingly, the method of the present invention may further comprise step (v): adding a volume greater than to the supernatant to the suspension culture. Vice versa, by adding a volume smaller than the volume of the supernatant to the suspension culture, the (total) volume of the suspension culture is decreased. Accordingly, the method of the present invention may further comprise step (v): adding a volume smaller than to the supernatant to the suspension culture.

The term "suspension culture" as used herein is a type of cell culture in which single cells or small aggregates of cells are allowed to function and multiply in an preferably agitated growth medium, thus forming a suspension (c.f. the definition in chemistry: "small solid particles suspended in a liquid"). This is in contrast to adherent culture, in which the cells are attached to a cell culture container, which may be coated with proteins of the extracellular matrix (ECM). In suspension culture, preferably no proteins of the ECM are added to the cells and/or the culture medium. The suspension culture preferably is essentially free of solid particles such as beads, microspheres, microcarrier particles and the like; cells or cell aggregates are no solid particles within this context. In one embodiment, the cells are not in microcarrier (suspension) culture.

The method of the present invention can also be used for medium exchange in microcarrier culture, i.e. for cells grown on microcarrier particles. Accordingly, the cells may be grown on microcarrier particles. "Microcarrier particles" as used herein relate to a support matrix allowing for the growth of adherent cells in bioreactors. Microcarriers are typically 125 - 250 µm spheres and their density allows them to be maintained in suspension with gentle stirring. Microcarriers can be made from a number of different materials including DEAE-dextran, glass, polystyrene plastic, acrylamide, collagen, and alginate. Surface chemistries can include extracellular matrix proteins, recombinant proteins, peptides, and positively or negatively charged molecules. Several types of microcarriers are available commercially including alginate-based (GEM, Global Cell Solutions), dextran-based (Cytodex, GE Healthcare), collagen-based (Cultispher, Percell), and polystyrene-based (SoloHill Engineering) microcarriers. They may differ in their porosity, specific gravity, optical properties, presence of animal components, and surface chemistries.

A "growth medium" or "culture medium" as used herein is a liquid designed to support the growth of microorganisms, cells, or small plants. Different types of media are used for growing different types of cells. A person skilled in the art is able to determine which culture medium is optimal for a specific cell type.

The method of the invention is preferably carried out in a bioreactor. As used herein, the terms "reactor" and "bioreactor", which can be used interchangeably, refer to a closed culture vessel configured to provide a dynamic fluid environment for cell cultivation. The bioreactor may be stirred and/or agitated. Examples of agitated reactors include, but are not limited to, stirred tank bioreactors, wave-mixed/rocking bioreactors, up and down agitation bioreactors (i.e., agitation reactor comprising piston action), spinner flasks, shaker flasks, shaken bioreactors, paddle mixers, vertical wheel bioreactors. An agitated reactor may be configured to house a cell culture volume of between about 2 mL - 20,000 L. Preferred bioreactors may have a volume of up to 50 L. An exemplary bioreactor suitable for the method of the present invention is the ambr15 bioreactor available from Sartorius Stedim Biotech. The bioreactor can be a stainless steel or a single use bioreactor. The bioreactor can consist of a single vessel or can comprise several bioreactors in parallel. The single use bioreactor can be manufactured from glass or plastic. The single use bioreactor can be a stirred tank bioreactor or a rocking motion bioreactor. Examples: Sartorius STR, RM, ambr15, ambr 250. The pH of the culture medium may be controlled by the bioreactor, preferably controlled by CO₂ supply, and may be held in a range of 6.6 to 7.6, preferably at about 7.4.

The bioreactor may be a stirred bioreactor (STR). STRs are, e.g., available from Sartorius Stedim Biotech and include, but are not limited to, BIOSTAT® A/B/B-DCU/Cplus/D-DCU, ambr® 15 and ambr® 250. The bioreactor may be a rocking motion bioreactor (RM). RMs are, e.g., available from Sartorius Stedim Biotech and include, but are not limited to, BIOSTAT® RM and BIOSTAT® RM TX. The bioreactor may be a multi parallel bioreactor that is, e.g., available from Sartorius Stedim Biotech and include, but are not limited to, ambr® 15 and ambr® 250.

In some embodiments, the volume of the culture vessel in the bioreactor is from about 50 mL to about 20,000 L. In some embodiments, the volume of the culture vessel in the bioreactor is from about 50 mL to about 2,000 L. In some embodiments, the volume of the culture vessel in the bioreactor is from about 50 mL to about 200 L. In some embodiments, the volume of the culture vessel in the bioreactor is from about 50 mL to about 100 L. In some embodiments, the volume of the culture vessel in the bioreactor is from about 50 mL to about 50 L. In some embodiments, the volume of the culture vessel in the bioreactor is from about 50 mL to about 20 L. In some embodiments, the volume of the culture vessel in the bioreactor is from about 50 mL to about 10 L. In some embodiments, the volume of the culture vessel in the bioreactor is from about 50 mL to about 1 L. In some embodiments, the volume of the culture vessel in the bioreactor is from about 100 mL to about 10 L. In some embodiments, the volume of the culture vessel in the bioreactor is from about 100 mL to about 5 L. In some embodiments, the volume of the culture vessel in the bioreactor is from about 150 mL to about 1 L. In some embodiments, the volume of the culture vessel in the bioreactor is from about 1 L to about 1,000 L.

As outlined herein, the cells in the suspension culture are preferably not sedimented but distributed in the culture medium. Accordingly, the suspension culture preferably is stirred. Continuous stirring may lead to an essentially homogenous distribution of the cells in the culture medium/suspension culture and may help stem cells such as iPSCs to maintain their pluripotency (see also Example 1 and Fig. 4). Accordingly, the cells preferably are essentially homogenously distributed in the culture medium.

The term "container" as used herein relates to any container that is suitable for retaining a suspension culture, the suspension culture comprising cells suspended in the culture medium. The container may be tubular, i.e. may have the form of a cylinder. One base of the cylinder is preferably essentially facing towards the gravitational force. Instead of a planar base, the container or the cylinder may have a conical bottom. The at least one opening may be at the very bottom of the conical surface. Such a container with conical bottom may also be described as a pipette tip. Thus, any type of pipette tips is envisioned as containers of the present invention.

The container is however not limited to pipette tips or the like. The container may also be a bag. A "bag" as used herein relates to a flexible container, preferably made from plastic, that comprises at least one flexible tube. The flexible tube may be immersed into the suspension culture, i.e. the "bottom" of the container may in case the container is a bag relate to the end of the tube that is immersed into the suspension culture. The tube may have a conical or conically-shaped end, which may be immersed into the suspension culture and corresponds to the bottom of the container described herein. Examples for a bag include, but are not limited to, single-use bags that may be commercially available from Sartorius Stedim biotech and sold as Flexsafe® 2D or Flexsafe® 3D bags. The bags may be for single-use.

To allow dispensing sedimented cells from the container back into the suspension culture, the container comprises at least one opening at the bottom through which the cell can be dispended. The fraction of the cells may be transferred into the container via the at least one opening at the bottom. It is however also envisioned by this invention that a different opening is used for the transfer of the fraction of the suspension culture into the container. If a (closed) bioreactor is used, the container is preferably inside the bioreactor.

The transfer of the fraction of the suspension culture may be performed by any suitable mean. E.g., the transfer is controlled by aspiration, e.g. by applying a slight negative pressure that aspirates the fraction into the container. Accordingly, the fraction of the suspension culture may be aspirated into the container in step (i).

The method of the present invention can generally be used for any cell that can be cultivated in cell culture, i.e. also for adherent cell culture. Advantageously, the method is used for changing culture medium of a suspension culture, in which the separation of the cells from the culture medium is of essence. In this context, "suspended in the culture medium" refers to cells that are cultured in suspension regardless if they actually are suspension cells or not. Thus, the method of the present invention can also be used for adherent cells, if they are suspended in the culture medium. Accordingly, the cells may by adherent cells that are cultured in suspension.

Adherent cells that are cultured in suspension, i.e. cannot attach to the culture vessel, may form cell aggregates. As used herein, the terms "aggregate" and "cell aggregate", which may be used interchangeably, refer to a plurality of cells such as (induced) pluripotent stem cells, in which an association between the cells is caused by cell-cell interaction (e.g., by biologic attachments to one another). Biological attachment may be, for example, through surface proteins, such integrins, immunoglobulins, cadherins, selectins, or other cell adhesion molecules. For example, cells may spontaneously associate in suspension and form cell-cell attachments (e.g., self-assembly), thereby forming aggregates. In some embodiments, a cell aggregate may be substantially homogeneous (i.e., mostly containing cells of the same type). In other embodiments, a cell aggregate may be heterogeneous, (i.e., containing cells of more than one type).

The method of the invention is suitable for cell aggregates. The cell aggregates may vary in size. The cell aggregates may have an average diameter between about 50 and 800 µm, between about 150 and 800 µm, of at least about 800 µm, of at least about 600 µm, of at least about 500 µm, of at least about 400 µm, of at least about 300 µm, of at least about 200 µm, of at least about 150 µm, between about 300 and 500 µm, between about 150 and 300 µm, between about 50 and 150 µm, between about 80 to 100 µm, between about 180 to 250 µm or between about 200 to 250 µm.

The cells may be any cells that can be cultivated in suspension, for example, prokaryotic cells or eukaryotic cells, with eukaryotic cells being preferred in one embodiment. The cells may be selected from the group consisting of, primary cells, cells obtained from a tissue or an organ, immortalized cells, pluripotent stem cells, preferably the cells are pluripotent stem cells, more preferably induced pluripotent stem cells (iPSCs), or cells derived from iPSCs. "Cells derived from iPSCs" relate to differentiated cells or cells differentiated into a specific cell type that are no longer capable of differentiating in any cell type of the body. Methods for the differentiation into different cell types starting from iPSCs are known to a person skilled in the art. "Cells derived from iPSCs" may relate to heart cells and/or tissue, liver cells and/or tissue, kidney cells and/or tissue, brain cells and/or tissue, pancreatic cells and/or tissue, lung cells and/or tissue, skeletal muscle cells and/or tissue, gastrointestinal cells and/or tissue, neuronal cells and/or tissue, skin cells and/or tissue, bone cells and/or tissue, bone marrow, fat cells and/or tissue, connective cells and/or tissue, retinal cells and/or tissue, blood vessel cells and/or tissue, stromal cells or cardiomyocytes. Methods for generating heart tissue are known from WO 2015/025030 and WO 2015/040142. The cells may also be differentiated in the bioreactor or also outside of the bioreactor, e.g. to cardiomyocytes or stromal cells. These differentiated cells may also be cultured in a bioreactor making use of the method of the invention. Cells obtained from a tissue or an organ may be obtained from heart cells and/or tissue, liver cells and/or tissue, kidney cells and/or tissue, brain cells and/or tissue, pancreatic cells and/or tissue, lung cells and/or tissue, skeletal muscle cells and/or tissue, gastrointestinal cells and/or tissue, neuronal cells and/or tissue, skin cells and/or tissue, bone cells and/or tissue, bone marrow, fat cells and/or tissue, connective cells and/or tissue, retinal cells and/or tissue, blood vessel cells and/or tissue, stromal cells or cardiomyocytes.

The cells may be cells of a mammal, such as a human, a dog, a mouse, a rat, a pig, an ape such as cynomolgus monkeys to name only a few illustrative examples. Preferably, the cells are human. Other preferred cells include fungal cells such as yeast cells such as P. *pastoris,* insect cells such as *Drosophila melanogaster* S2 and *Spodoptera frugiperda* Sf9 cells, bacterial cells such as *E. coli, Streptomyces* and *Salmonella typhimurium* cells, or plant cells.

The term "pluripotent stem cell" (PSC) as used herein refers to cells that are able to differentiate into every cell type of the body. As such, pluripotent stem cells offer the unique opportunity to be differentiated into essentially any tissue or organ. Currently, the most utilized pluripotent cells are embryonic stem cells (ESC) or induced pluripotent stem cells (iPSC). Human ESC-lines were first established by Thomson and coworkers (Thomson et al. (1998), Science 282:1145-1147). Human ESC research recently enabled the development of a new technology to reprogram cells of the body into an ES-like cell. This technology was pioneered by Yamanaka and coworkers in 2006 (Takahashi & Yamanaka (2006), Cell, 126:663-676). Resulting induced pluripotent cells (iPSC) show a very similar behavior as ESC and, importantly, are also able to differentiate into every cell of the body. Thus, in one embodiment, the term iPSCs comprises ESC. In the context of the present invention, these pluripotent stem cells are however preferably not produced using a process which involves modifying the germ line genetic identity of human beings or which involves use of a human embryo for industrial or commercial purposes. Preferably, the pluripotent stem cells are of primate origin, more preferably human.

Suitable induced PSCs, can for example, be obtained from the NIH human embryonic stem cell registry, the European Bank of Induced Pluripotent Stem Cells (EBiSC), the Stem Cell Repository of the German Center for Cardiovascular Research (DZHK), or ATCC, to name only a few sources. Induced pluripotent stem cells are also available for commercial use, for example, from the NINDS Human Sequence and Cell Repository (https://stemcells.nindsgenetics.org) which is operated by the U.S. National Institute of Neurological Disorders and Stroke (NINDS) and distributes human cell resources broadly to academic and industry researchers. One illustrative example of a suitable cell line that can be used in the present invention is the cell line TC-1133, an induced (unedited) pluripotent stem cell that has been derived from a cord blood stem cell. This cell line is, e.g. directly available from NINDS, USA. Preferably, TC-1133 is GMP-compliant. Further exemplary iPSC cell lines that can be used in the present invention, include but are not limited to, the Human Episomal iPSC Line of Gibco™ (order number A18945, Thermo Fisher Scientific), or the iPSC cell lines ATCC ACS-1004, ATCC ACS-1021, ATCC ACS-1025, ATCC ACS-1027 or ATCC ACS-1030 available from ATTC. Alternatively, any person skilled in the art of reprogramming can easily generate suitable iPSC lines by known protocols such as the one described by Okita et al, "A more efficient method to generate integration-free human iPS cells" Nature Methods, Vol.8 No.5, May 2011, pages 409-411 or by Lu et al "A defined xeno-free and feeder-free culture system for the derivation, expansion and direct differentiation of transgene-free patient-specific induced pluripotent stem cells", Biomaterials 35 (2014) 2816e2826.

The cells may be selected from the group consisting of TC-1133, the Human Episomal iPSC Line of Gibco ATCC ACS-1004, ATCC ACS-1021, ATCC ACS-1025, ATCC ACS-1027, ATCC ACS-1030, HEK293, HEK293T, BHK 21, CHO, NS0, Sp2/0-Ag14.

As explained herein, the (induced) pluripotent stem cell that is used in the present invention can be derived from any suitable cell type (for example, from a stem cell such as a mesenchymal stem cell, or an epithelial stem cell or a differentiated cells such as fibroblasts) and from any suitable source (bodily fluid or tissue). Examples of such sources (body fluids or tissue) include cord blood, skin, gingiva, urine, blood, bone marrow, any compartment of the umbilical cord (for example, the amniotic membrane of umbilical cord or Wharton's jelly), the cord-placenta junction, placenta or adipose tissue, to name only a few. In one illustrative example, is the isolation of CD34-positive cells from umbilical cord blood for example by magnetic cell sorting using antibodies specifically directed against CD34 followed by reprogramming as described in Chou et al. (2011), Cell Research, 21:518-529. Baghbaderani et al. (2015), Stem Cell Reports, 5(4):647-659 show that the process of iPSC generation can be in compliance with the regulations of good manufacturing practice to generate cell line ND50039.

Accordingly, the pluripotent stem cell preferably fulfils the requirements of the good manufacturing practice.

The method of the invention as outlined herein requires the sedimentation of the suspended cells in step (ii). The time needed for sedimentation may vary with the size, form and mass of the cells. It is within the knowledge of a person skilled in the art to determine the time necessary for a sufficient sedimentation of the cells. Accordingly, in step (ii) of the method of the invention, the period, in which the cells are allowed to settle, can be at least 1 min, at least 2 min, at least 3 min, at least 4 min, at least 5 min, at least 6 min, at least 7 min, at least 8 min, at least 9 min, at least 10 min, at least 11 min, at least 12 min, at least 13 min, at least 14 min, at least 15 min, at least 16 min, at least 17 min, at least 18 min, at least 19 min or at least 20 min.

"Sufficient sedimentation" means that at least 70%, at least 80%, at least 90%, at least 95%, at least 97.5%, at least 99% or essentially all cells of the cells transferred in step (i) are dispensed (back) into the suspension culture in step (iii). Accordingly, at least 70%, at least 80%, at least 90%, at least 95%, at least 97.5%, at least 99% or essentially all cells of the cells transferred in step (i) are preferably dispensed (back) into the suspension culture in step (iii).

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

As used herein the terms "about", "approximately" or "essentially" mean within 20%, preferably within 15%, preferably within 10%, and more preferably within 5% of a given value or range. It also includes the concrete number, i.e. "about 20" includes the number of 20.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

### EXAMPLES

An even better understanding of the present invention and of its advantages will be evident from the following examples, offered for illustrative purposes only. The examples are not intended to limit the scope of the present invention in any way.

### Materials and Methods

The following materials and methods were used throughout the example unless specified otherwise.

### Cell culture

- Cells: TC1133, NINDS
- Seeding density: 5x10⁵ cells / mL
- Culture conditions: 37°C, pH 7.4, dO 50%, 300rpm downstirr.
- Beginning of medium exchange on day 2.

### Materials

- ambr15 cell culture 24 Disposable bioreactors, Low temp, no sparger, Part number: 001-2B81
- StemMACS iPS-Brew XF, Basal Medium, Order no.: 130-107-086
- StemMACS ™ iPS-Brew XF 50x Supplement; Order no.: 130-107-087
- Ambr15 bioreactor, Sartorius Stedim
- Nucleocounter NC-200 Type 900-0201
- Cellavista Cell Imager
- Flow Cytometer: BD LSR II Special Order System

### Medium exchange

### Vessel settling:

1. Stop stirring of culture station
2. Pause for 5min to let aggregate settle to the bottom
3. Aspiration of medium with a distance between pipette tip and vessel bottom of 1.4 cm
4. Addition of fresh medium
5. Start stirring of culture station
6. Overall exchange of medium: 66 %

### Tip settling:

1. Uptake of 900 µl cell suspension into the pipette tip
2. Pause for 5min with suspension remaining in tip
3. Return of 100µl into the vessel. Discarding of remaining spent medium.
4. Addition of 800 µl fresh medium into the vessel
5. 6 repetitions of Step 1-4
6. Overall exchange of medium: 57%

### Example 1: Assessment of the vessel settling strategy and tip settling strategy as means for medium exchange of iPSC culture in the ambr15 system at maximum capacity

The inventors compared two medium exchange strategies for iPSC culture in the ambr15 at maximum capacity of 24 vessels. To test both strategies in parallel, the vessel settling strategy was performed in culture station 1 and the tip settling strategy was performed in culture station 2. The maximum capacity of the ambr15 system was mimicked as followed: Repeated intervals of activated stirring (860 seconds; simulating medium exchange of a vessel of the opposite culture station) and deactivated stirring (881 seconds; simulating exchange of vessels in the same culture station) were included during vessel setting. The durations were empirically determined previously. A pause of 2.5 h was included between each interval of tip settling medium exchange.

### Results

### Morphology

The morphology of iPSC aggregates was comparable before initiating medium exchange (Figure 2). After medium exchange the aggregates of the vessel settling strategy had diffuse or tubular shape and were large. This observation indicates fusion of aggregates. In contrast, aggregates of tip settling strategy were round and small.

### Cell number and expansion rates

The cell number after 4 days of suspension culture increased 3.61-fold using the vessel settling strategy. The tip settling strategy resulted in a mean increase of 21.81-fold during the same time period (Figure 3).

### Pluripotency

The expression of pluripotency-related genes (OCT4, TRA-1-60, NANOG) was high after four days of suspension culture using the tip settling strategy (90% OCT4/TRA-1-60 double positive cells, 91% OCT4/NANOG double positive cells, Fig. 4). The expression was considerably lower in cells cultured with the vessel settling strategy (11% OCT4/TRA-1-60 double positive cells, 9% OCT4/NANOG double positive cells, Fig. 4).

### Analysis

The tip settling strategy is more suitable for medium exchange of cell culture, in particular of iPSCs, in bioreactors such as the ambr15 system at maximum capacity compared to the vessel settling strategy. Considering the poor expression of pluripotency-related markers, the vessel settling strategy may even be completely unfit for medium exchange, in particular in the ambr15 at maximum capacity of 24 vessels. The reason for this likely is the fusion of aggregates during time periods without agitation resulting in spontaneous differentiation.

### REFERENCES

Amit, Michal; Chebath, Judith; Margulets, Victoria; Laevsky, Ilana; Miropolsky, Yael; Shariki, Kohava et al. (2010): Suspension culture of undifferentiated human embryonic and induced pluripotent stem cells. In: Stem cell reviews 6 (2), S. 248-259.
Kwok, Chee Keong; Ueda, Yuichiro; Kadari, Asifiqbal; Günther, Katharina; Ergün, Süleyman; Heron, Antoine et al. (2018): Scalable stirred suspension culture for the generation of billions of human induced pluripotent stem cells using single-use bioreactors. In: Journal of tissue engineering and regenerative medicine 12 (2), e1076-e1087. DOI: 10.1002/term.2435.
Lipsitz, Yonatan Y.; Tonge, Peter D.; Zandstra, Peter W. (2018): Chemically controlled aggregation of pluripotent stem cells. In: Biotechnology and bioengineering 115 (8), S. 2061-2066. DOI: 10.1002/bit.26719.
Olmer, Ruth; Lange, Andreas; Selzer, Sebastian; Kasper, Cornelia; Haverich, Axel; Martin, Ulrich; Zweigerdt, Robert (2012): Suspension Culture of Human Pluripotent Stem Cells in Controlled, Stirred Bioreactors. In: Tissue engineering. Part C, Methods 18 (10), S. 772-784. DOI: 10.1089/ten.tec.2011.0717.

## Claims

1. A method of changing culture medium of a suspension culture, the suspension culture comprising cells suspended in the culture medium, the method comprising:
(i) transferring a fraction of the suspension culture into a container, wherein the container comprises at least one opening at the bottom;
(ii) allowing the cells comprised in the fraction of the suspension to settle at the at least one opening at the bottom of the container by gravitation, thereby forming a supernatant;
(iii) dispensing the cells settled at the bottom of the container (back) into the suspension culture;
(iv) discarding the supernatant.

2. The method of any one of the preceding claims, wherein the method is carried out in a bioreactor, wherein the bioreactor preferably is a stirred bioreactor, a rocking motion bioreactor and/or a multi parallel bioreactor.

3. The method of any one of the preceding claims, wherein the suspension culture is continuously stirred;
and/or wherein the cells are essentially homogenously distributed in the culture medium.

4. The method of any one of the preceding claims, wherein the container is tubular; and/or wherein the container has a conical bottom; and/or
wherein the container is a pipette tip, a (single-use) bag or the conical/conically-shaped part of a (single-use) bag.

5. The method of any one of the preceding claims, wherein the fraction of the suspension culture is aspirated into the container in step (i).

6. The method of any one of the preceding claims, wherein the cells are eukaryotic cells such as human cells, fungal cells such as yeast cells such as P. *pastoris,* insect cells such as *Drosophila melanogaster* S2 and *Spodoptera frugiperda* Sf9 cells, bacterial cells such as *E. coli, Streptomyces* and *Salmonella typhimurium* cells, or plant cells.

7. The method of any one of the preceding claims, wherein the cells are adherent cells that are cultured in suspension.

8. The method of any one of the preceding claims, wherein the cells are selected from the group consisting of, primary cells, cells obtained from a tissue or an organ, immortalized cells, pluripotent stem cells, preferably the cells are pluripotent stem cells, more preferably induced pluripotent stem cells (iPSC).

9. The method of any one of the preceding claims, wherein the cells are selected from the group consisting of TC-1133, the Human Episomal iPSC Line of Gibco ATCC ACS-1004, ATCC ACS-1021, ATCC ACS-1025, ATCC ACS-1027, ATCC ACS-1030, HEK293, HEK293T, BHK 21, CHO, NS0, Sp2/0-Ag14.

10. The method of any one of the preceding claims, wherein the cells are human.

11. The method of any one of the preceding claims, wherein the cells are cell aggregates, wherein the cell aggregates preferably have an average diameter between about 50 and 800 µm, between about 150 and 800 µm, of at least about 800 µm, of at least about 600 µm, of at least about 500 µm, of at least about 400 µm, of at least about 300 µm, of at least about 200 µm, of at least about 150 µm, between about 300 and 500 µm, between about 150 and 300 µm, between about 50 and 150 µm, between about 80 to 100 µm, between about 180 to 250 µm or between about 200 to 250 µm.

12. The method of any one of the preceding further comprising
(v) adding a volume equivalent to the supernatant to the suspension culture.

13. The method of any one of the preceding claims, wherein in step (ii) the period, in which the cells are allowed to settle, is at least 1 min, at least 2 min, at least 3 min, at least 4 min, at least 5 min, at least 6 min, at least 7 min, at least 8 min, at least 9 min, at least 10 min, at least 11 min, at least 12 min, at least 13 min, at least 14 min, at least 15 min, at least 16 min, at least 17 min, at least 18 min, at least 19 min or at least 20 min.

14. The method of any one of the preceding claims, wherein at least 70%, at least 80%, at least 90%, at least 95%, at least 97.5%, at least 99% or essentially all cells of the cells transferred in step (i) are dispensed (back) into the suspension culture in step (iii).

15. The method of any one of claims 1-7, 9-10, and 12-14, wherein the cells are grown on microcarrier particles and/or wherein the suspension culture is a microcarrier culture.
